(19) Europäisches Patentamt
European Patent Office
Office européen des brevets

(11) **EP 4 495 095 A1**

(12) **EUROPEAN PATENT APPLICATION**
published in accordance with Art. 153(4) EPC

(43) Date of publication:
    **22.01.2025 Bulletin 2025/04**

(21) Application number: **23770392.1**

(22) Date of filing: **28.02.2023**

(51) International Patent Classification (IPC):
    **C07C 17/38** (2006.01)     **C07C 21/18** (2006.01)

(52) Cooperative Patent Classification (CPC):
    **C07C 17/38; C07C 21/18**

(86) International application number:
    **PCT/JP2023/007363**

(87) International publication number:
    **WO 2023/176434 (21.09.2023 Gazette 2023/38)**

(84) Designated Contracting States:
    **AL AT BE BG CH CY CZ DE DK EE ES FI FR GB
    GR HR HU IE IS IT LI LT LU LV MC ME MK MT NL
    NO PL PT RO RS SE SI SK SM TR**
    Designated Extension States:
    **BA**
    Designated Validation States:
    **KH MA MD TN**

(30) Priority: **16.03.2022 JP 2022041526**

(71) Applicant: **Resonac Corporation
    Tokyo 105-7325 (JP)**

(72) Inventor: **OHTSUKI Kazuaki
    Tokyo 105-8518 (JP)**

(74) Representative: **Strehl Schübel-Hopf & Partner
    Maximilianstrasse 54
    80538 München (DE)**

(54) **METHOD FOR STORING FLUOROALKENE**

(57)    There is provided a method for storing fluoroalkene in which, even when stabilizers for suppressing reactions of fluoroalkene are not added, the reactions of the fluoroalkene hardly occur and the purity of the fluoroalkene hardly decreases during storage. Fluoroalkene having a bromine atom or an iodine atom in the molecule is at least one type of fluoroethylene represented by a first general formula $C_2H_pF_qX_r$ and fluoro-propene represented by a second general formula $C_3H_sF_tX_u$ (X represents a bromine atom or an iodine atom), and contains or does not contain at least one type among manganese, cobalt, nickel, and silicon as metal impurities. When any of the foregoing is contained, the fluoroalkene is stored in a container with the total concentration of the manganese, the cobalt, the nickel, and the silicon set to 1000 ppb by mass or less.

**EP 4 495 095 A1**

**Description**

Technical Field

The present invention relates to a method for storing fluoroalkene.

Background Art

Fluoroalkene having a bromine atom or an iodine atom in the molecule (e.g., 2-bromo-3,3,3-trifluoropropene) is sometimes used as a digester or an etching gas for dry etching (see PTLS 1, 2, for example). When "fluoroalkene" is simply described in this specification, it means "fluoroalkene having a bromine atom or an iodine atom in the molecule".

Citation List

Patent Literatures

PTL 1: JP 2014-534840 A
PTL 2: JP 2017-190311 A

Summary of Invention

Technical Problem

However, the fluoroalkene is a compound having insufficient stability, and therefore has posed a risk that a decomposition reaction and an elimination reaction of the bromine atom or the iodine atom progress, reducing the purity of the fluoroalkene. The reactions of the fluoroalkene can be suppressed by adding stabilizers, such as antioxidants and water, to the fluoroalkene. However, there has been such a problem that the addition of the stabilizers reduces the purity of the fluoroalkene.
It is an object of the present invention to provide a method for storing fluoroalkene in which the reactions of the fluoroalkene hardly occur during storage and the purity of the fluoroalkene hardly decreases without the addition of the stabilizers for suppressing the reactions of the fluoroalkene.

Solution to Problem

To achieve the above-described object, one aspect of the present invention is as described in [1] to [5] below.

[1] A method for storing fluoroalkene, the fluoroalkene having a bromine atom or an iodine atom in the molecule, including: storing the fluoroalkene in a container, in which

the fluoroalkene is at least one type of fluoroethylene represented by a first general formula $C_2H_pF_qX_r$ and, in the first general formula, X represents a bromine atom or an iodine atom, p is an integer of 0 or more and 2 or less, q is an integer of 1 or more and 3 or less, r is an integer of 1 or more and 3 or less, and p + q + r is 4, and fluoropropene represented by a second general formula $C_3H_sF_tX_u$ and, in the second general formula, X represents a bromine atom or an iodine atom, s is an integer of 0 or more and 4 or less, t is an integer of 1 or more and 5 or less, u is an integer of 1 or more and 5 or less, and s + t + u is 6, and
the fluoroalkene contains or does not contain at least one type among manganese, cobalt, nickel, and silicon as metal impurities, and, when any of the foregoing is contained, the total concentration of the manganese, the cobalt, the nickel, and the silicon is set to 1000 ppb by mass or less.

[2] The method for storing fluoroalkene according to [1], including: storing the fluoroalkene in a container, in which the fluoroalkene further contains or does not contain at least one type among sodium, potassium, magnesium, and calcium as the metal impurities, and, when any of the foregoing is further contained, the total concentration of the manganese, the cobalt, the nickel, and the silicon and the sodium, the potassium, the magnesium, and the calcium is set to 2000 ppb by mass or less.
[3] The method for storing fluoroalkene according to [1] or [2], in which the fluoroalkene is at least one selected from 1-bromo-1,2,2-trifluoroethylene, 1-bromo-1-fluoroethylene, 1-iodo-1,2,2-trifluoroethylene, 1-iodo-1-fluoroethylene, 2-bromo-3,3,3-trifluoropropene, and 2-iodo-3,3,3-trifluoropropene.

[4] The method for storing fluoroalkene according to any one of [1] to [3], including: storing the fluoroalkene at a temperature of -20°C or more and 50°C or less.

[5] The method for storing fluoroalkene according to any one of [1] to [4], in which a material of the container is manganese steel.

Advantageous Effects of Invention

According to the present invention, even when the stabilizers for suppressing the reactions of the fluoroalkene are not added, the reactions of the fluoroalkene hardly occur and the purity of the fluoroalkene hardly decreases during storage.

Description of Embodiments

Hereinafter, one embodiment of the present invention is described. This embodiment describes one example of the present invention, and the present invention is not limited to this embodiment. This embodiment can be variously altered or improved, and such altered or improved aspects can also be included in the present invention.

A method for storing fluoroalkene according to this embodiment is a method for storing fluoroalkene in which the fluoroalkene has a bromine atom or an iodine atom in the molecule, and includes storing the fluoroalkene in a container, in which the fluoroalkene contains or does not contain at least one type among manganese (Mn), cobalt (Co), nickel (Ni), and silicon (Si) as metal impurities, and, when any of the foregoing is contained, the total concentration of the manganese, the cobalt, the nickel, and the silicon is set to 1000 ppb by mass or less.

The fluoroalkene is at least one type of fluoroethylene and fluoropropene. The fluoroethylene is a compound represented by a first general formula $C_2H_pF_qX_r$ and, in the first general formula, X represents a bromine atom or an iodine atom, p is an integer of 0 or more and 2 or less, q is an integer of 1 or more and 3 or less, r is an integer of 1 or more and 3 or less, and p + q + r is 4. The fluoropropene is a compound represented by a second general formula $C_3H_sF_tX_u$ and, in the second general formula, X represents a bromine atom or an iodine atom, s is an integer of 0 or more and 4 or less, t is an integer of 1 or more and 5 or less, u is an integer of 1 or more and 5 or less, and s + t + u is 6.

When the fluoroalkene contains at least one type among manganese, cobalt, nickel, and silicon as metal impurities, the catalysis of the metal impurities accelerates a decomposition reaction of the fluoroalkene or an elimination reaction of the bromine atom or the iodine atom from the fluoroalkene, for example. Therefore, the fluoroalkene containing the metal impurities is likely to generate decomposition products of the fluoroalkene, bromine molecules, or iodine molecules during long-term storage, for example, and is likely to decrease in purity.

The fluoroalkene stored according to the method for storing fluoroalkene of this embodiment does not contain the metal impurities or, even when the fluoroalkene contains the metal impurities, the content is very small. Therefore, even in long-term storage, for example, the decomposition reaction of the fluoroalkene and the elimination reaction of the bromine atom or the iodine atom from the fluoroalkene hardly progress and decomposition products of the fluoroalkene, bromine molecules, or iodine molecules are hardly generated. Therefore, the decrease in purity of the fluoroalkene hardly occurs, and the high purity can be maintained over a long period of time.

Thus, the fluoroalkene can be stably stored over a long period of time without adding stabilizers, such as antioxidants and water, to the fluoroalkene. Further, the decrease in purity of the fluoroalkene by the addition of the stabilizers does not occur and the decrease in quality (e.g., quality as a digester or an etching gas) of the fluoroalkene by the addition of the stabilizers does not occur. In the method for storing fluoroalkene according to this embodiment, the stabilizers may be added to the fluoroalkene. However, a preferable aspect of the method for storing fluoroalkene according to this embodiment is an aspect in which no stabilizers are added to the fluoroalkene.

Hereinafter, the method for storing fluoroalkene according to this embodiment is described in more detail.
[Fluoroalkene having bromine atom or iodine atom in molecule]

The fluoroalkene in the method for storing fluoroalkene according to this embodiment is at least one type of the fluoroethylene represented by the first general formula $C_2H_pF_qX_r$ above and the fluoropropene represented by the second general formula $C_3H_sF_tX_u$ above. The type of the fluoroalkene is not particularly limited insofar as it satisfies the above-described requirements, and fluoroalkene where r is 1 in the first general formula above and fluoroalkene where u is 1 in the second general formula above are preferable.

Specific examples of the fluoroalkene having a bromine atom in the molecule include $CFBr=CH_2$(1-bromo-1-fluoroethylene), $CFBr=CHF$, $CFBr=CF_2$(1-bromo-1,2,2-trifluoroethylene), $CFBr=CFBr$, $CFBr=CHBr$, $CF_2=CHBr$, $CHF=CHBr$, $CFBr=CBr_2$, $CF_2=CBr_2$, $CHF=CBr_2$, $CHBr=CBr_2$, $CH_2=CBrCF_3$(2-bromo-3,3,3-trifluoropropene), $CHF=CBrCF_3$, $CHBr=CBrCF_3$, $CF_2=CBrCF_3$, $CBr_2=CBrCF_3$, $CFBr=CBrCF_3$, $CH_2=CBrCF_2Br$, $CHF=CBrCF_2Br$, $CHBr=CBrCF_2Br$, $CF_2=CBrCF_2Br$, $CBr_2=CBrCF_2Br$, $CFBr=CBrCF_2Br$, $CH_2=CBrCFBr_2$, $CHF=CBrCFBr_2$, $CHBr=CBrCFBr_2$, and $CF_2=CBrCFBr_2$.

Specific examples of the fluoroalkene having an iodine atom in the molecule include $CFI=CH_2$(1-iodo-1-fluoroethylene), $CFI=CHF$, $CFI=CF_2$(1-iodo-1,2,2-trifluoroethylene), $CFI=CFI$, $CFI=CHI$, $CF_2=CHI$, $CHF=CHI$, $CFI=CI_2$, $CF_2=CI_2$, $CHF=CI_2$, $CHI=CI_2$, $CH_2=CICF_3$(2-iodo-3,3,3-trifluoropropene), $CHF=CICF_3$, $CHI=CICF_3$, $CF_2=CICF_3$, $CI_2=CICF_3$, $CFI=CICF_3$, $CH_2=CICF_2I$, $CHF=CICF_2I$, $CHI=CICF_2I$, $CF_2=CICF_2I$, $CI_2=CICF_2I$, $CFI=CICF_2I$, $CH_2=CICFI_2$, $CHF=CICFI_2$, $CHI=CICFI_2$, and $CF_2=CICFI_2$.

These fluoroalkenes may be used alone or two or more types in combination. Some of the above-described fluoroalkenes have cis-trans isomers, and both cis and trans fluoroalkenes are usable for the method for storing fluoroalkene according to this embodiment.

When the fluoroalkene is stored in a container, gas containing only the fluoroalkene may be stored in the container or mixed gas containing the fluoroalkene and inert dilution gas may be stored in the container. The fluoroalkene may also be partially or entirely liquefied and stored in the container. As the dilution gas, at least one type selected from nitrogen gas ($N_2$), helium (He), neon (Ne), argon (Ar), krypton (Kr), and xenon (Xe) is usable. The content of the dilution gas is preferably 90% by volume or less and more preferably 50% by volume or less based on the total amount of the gas stored in the container.

[Container]

The container in which the fluoroalkene is stored is not particularly limited in shape, size, material, and the like insofar as it is capable of housing and sealing the fluoroalkene. As the material of the container, metal, ceramic, resin, and the like can be adopted. Examples of the metal include manganese steel, stainless steel, Hastelloy (registered trademark), Inconel (registered trademark), and the like.

[Metal impurities]

The fluoroalkene in the method for storing fluoroalkene according to this embodiment contains or does not contain at least one type among manganese, cobalt, nickel, and silicon as the metal impurities, and, when any of the foregoing is contained, the fluoroalkene is stored in the container with the total concentration of the manganese, the cobalt, the nickel, and the silicon set to 1000 ppb by mass or less, and therefore the above-described effects are exhibited. More specifically, the decomposition reaction of the fluoroalkene and the elimination reaction of the bromine atom and the iodine atom from the fluoroalkene hardly progress, and decomposition products of the fluoroalkene, bromine molecules, and iodine molecules are hardly generated.

Herein, not containing the metal impurities refers to a case where the quantification with an inductively coupled plasma mass spectrometer (ICP-MS) is impossible. The metals, such as manganese, cobalt, nickel, and silicon, in the present invention include metal atoms and metal ions.

To suppress the progress of the decomposition reaction of the fluoroalkene and the elimination reaction of the bromine atom or the iodine atom from the fluoroalkene during storage, the total concentration of the manganese, the cobalt, the nickel, and the silicon contained in the fluoroalkene is required to be 1000 ppb by mass or less, preferably 500 ppb by mass or less, and more preferably 100 ppb by mass or less.

To further suppress the above-described decomposition reaction and the above-described elimination reaction during storage, the concentration of each of the manganese, the cobalt, the nickel, and the silicon contained in fluoroalkene is preferably 300 ppb by mass or less and more preferably 100 ppb by mass or less.

The total concentration of the manganese, the cobalt, the nickel, and the silicon may be 1 ppb by mass or more.

The concentrations of the metal impurities, such as the manganese, the cobalt, the nickel, and the silicon, in the fluoroalkene can be quantified with an inductively coupled plasma mass spectrometer (ICP-MS).

To further suppress the above-described decomposition reaction and the above-described elimination reaction during storage, the concentrations of sodium (Na), potassium (K), magnesium (Mg), and calcium (Ca) are preferably set to be low as well as the concentrations of the manganese, the cobalt, the nickel, and the silicon in the fluoroalkene.

More specifically, the fluoroalkene is preferably stored as follows. The fluoroalkene contains or does not contain at least one type among manganese, cobalt, nickel, and silicon as the metal impurities and, when any of the foregoing is contained, the total concentration of the manganese, the cobalt, the nickel, and the silicon is set to 1000 ppb by mass or less. In addition, the fluoroalkene further contains or does not contain at least one type among sodium, potassium, magnesium, and calcium as the metal impurities, and, when any of the foregoing is further contained, the total concentration of the manganese, the cobalt, the nickel, and the silicon and the sodium, the potassium, the magnesium, and the calcium is set to 2000 ppb by mass or less.

When any of the foregoing is contained, the total concentration of the manganese, the cobalt, the nickel, and the silicon and the sodium, the potassium, the magnesium, and the calcium is preferably set to 1000 ppb by mass or less and more preferably set to 500 ppb by mass or less.

The total concentration of the manganese, the cobalt, the nickel, and the silicon and the sodium, the potassium, the magnesium, and the calcium may be 2 ppb by mass or more.

To further suppress the above-described elimination reaction during storage, the concentrations of copper (Cu), zinc (Zn), and aluminum (Al), as well as the concentrations of the manganese, the cobalt, the nickel, and the silicon and the concentrations of the sodium, the potassium, the magnesium, and the calcium in the fluoroalkene, are preferably set to be low.

More specifically, when the fluoroalkene contains at least one type among manganese, cobalt, nickel, and silicon and at least one type among sodium, potassium, magnesium, and calcium as the metal impurities, and further contains at least one type among copper, zinc, and aluminum as the metal impurities, the fluoroalkene is preferably stored with the total concentration of all these contained metal impurities set to 3000 ppb by mass or less. The total concentration of all these contained metal impurities is more preferably set to 1500 ppb by mass or less and still more preferably set to 1000 ppb by mass or less.

The above-described metal impurities are sometimes contained in the fluoroalkene in the forms of a metal simple substance, a metal compound, a metal halide, and a metal complex. The forms of the metal impurities in the fluoroalkene include fine particles, liquid droplets, gas, and the like. The manganese, the cobalt, the nickel, and the silicon are considered to be mixed into the fluoroalkene originating from raw materials, reactors, refining devices, and the like used in the synthesis of the fluoroalkene.

[Method for manufacturing fluoroalkene having low concentration of metal impurities]

A method for manufacturing fluoroalkene having a low concentration of metal impurities is not particularly limited, and includes a method for removing metal impurities from fluoroalkene having a high concentration of metal impurities, for example. A method for removing metal impurities from fluoroalkene is not particularly limited, and known methods can be adopted. Examples include a method using a filter, a method using an adsorbent, and distillation.

A material of a filter, through which fluoroalkene gas is selectively passed, is preferably resin to avoid the mixing of metal components into the fluoroalkene and is particularly preferably polytetrafluoroethylene. The average pore size of the filter is preferably 0.01 μm or more and 30 μm or less and more preferably 0.1 μm or more and 10 μm or less. When the average pore size is in the ranges above, the metal impurities can be sufficiently removed, and a sufficient flow rate of the fluoroalkene gas can be ensured and high productivity can be achieved.

The flow rate at which the fluoroalkene gas is passed through a filter is preferably set to 3 mL/min or more and 300 mL/min or less and more preferably set to 10 mL/min or more and 50 mL/min or less per $cm^2$ of the filter area. When the flow rate of the fluoroalkene gas is in the ranges above, the pressure of the fluoroalkene gas is prevented from becoming high, so that a risk of leakage of the fluoroalkene gas is lowered and high productivity can be achieved.

[Pressure condition in storage]

A pressure condition in storage in the method for storing fluoroalkene according to this embodiment is not particularly limited insofar as the fluoroalkene can be sealed and stored in a container, and is preferably set to 0.01 MPa or more and 5 MPa or less and more preferably set to 0.05 MPa or more and 3 MPa or less. The pressure condition in the ranges above makes it possible to make the fluoroalkene flow without being humidified when the container is connected to a dry etching device.

[Temperature condition in storage]

A temperature condition in storage in the method for storing fluoroalkene according to this embodiment is not particularly limited, and is preferably set to -20°C or more and 50°C or less and more preferably set to 0°C or more and 40°C or less. When the temperature in storage is -20°C or more, the container is hardly deformed, and therefore a possibility that the airtightness of the container is lost, allowing the mixing of oxygen, water, and the like into the container, is low. The mixing of oxygen, water, and the like poses a risk that a polymerization reaction and a decomposition reaction of the fluoroalkene are accelerated. When the temperature in storage is 50°C or less, a polymerization reaction and a decomposition reaction of the fluoroalkene are suppressed.

[Etching]

The fluoroalkene stored in the method for storing fluoroalkene according to this embodiment is usable as an etching gas. An etching gas containing the fluoroalkene stored according to the method for storing fluoroalkene of this embodiment is usable for both plasma etching using a plasma and plasma-less etching without using a plasma.

Examples of the plasma etching include reactive ion etching (RIE), inductively coupled plasma (ICP) etching, capacitively coupled plasma (CCP) etching, electron cyclotron resonance (ECR) plasma etching, and microwave plasma etching.

In the plasma etching, a plasma may be generated in a chamber where a material to be etched is placed or a plasma generating chamber and the chamber where a material to be etched is placed may be separated from each other (i.e., remote plasma may be used).

EXAMPLES

Hereinafter, the present invention is more specifically described with reference to Examples and Comparative Examples. Fluoroalkenes containing various concentrations of metal impurities were prepared. Preparation Examples of the fluoroalkenes are described below.

(Preparation Example 1)

One 10 L volume manganese steel cylinder and four 1 L volume sealable manganese steel cylinders were prepared. The cylinders are referred to as a cylinder A, a cylinder B, a cylinder C, and a cylinder D in order. The cylinder was filled with 5000 g of 1-bromo-1,2,2-trifluoroethylene. The 1-bromo-1,2,2-trifluoroethylene was liquefied by being cooled to 0°C, forming a liquid phase part and a gas phase part at about 100 kPa. The cylinders A, B, C, D were cooled to -78°C after the inside was depressurized to 1 kPa or less with a vacuum pump.

500 g of 1-bromo-1,2,2-trifluoroethylene gas was extracted from an upper outlet, where the gas phase part is present, of the cylinder, passed through a filter, and then liquefied at -78°C and collected in the cylinder A in the depressurized state. The filter is a PTFE filter manufactured by FLON INDUSTRY and has an outer diameter of 50 mm, a thickness of 80 $\mu$m, and an average pore size of 0.3 $\mu$m. The flow rate of the gas when passed through the filter was controlled to 500 mL/min with a mass flow controller. The amount of the 1-bromo-1,2,2-trifluoroethylene collected in the cylinder A was 492 g.

The 1-bromo-1,2,2-trifluoroethylene collected in the cylinder A is set as Sample 1-1. The 1-bromo-1,2,2-trifluoroethylene collected in the cylinder A has a gas phase part and a liquid phase part. The gas phase part was extracted from an upper outlet and the concentrations of various metal impurities were measured with an inductively coupled plasma mass spectrometer. The results are shown in Table 1.

The details of a method for measuring the concentrations of various metal impurities using an inductively coupled plasma mass spectrometer are as follows.

While the 1-bromo-1,2,2-trifluoroethylene in the liquid phase in the cylinder A was being vaporized at 20°C, the 1-bromo-1,2,2-trifluoroethylene gas was extracted from the gas phase part, and made to flow at a flow rate of 100 mL/min into 100 g of an aqueous nitric acid solution having a concentration of 1 mol/L for bubbling. This bubbling brought the 1-bromo-1,2,2-trifluoroethylene and the aqueous nitric acid solution into contact with each other, making the aqueous nitric acid solution absorb the metal impurities. The mass of the aqueous nitric acid solution after the bubbling was 80 g (M1). A mass difference in the cylinder A before and after the bubbling was 50 g (M2).

10 g (M3) of the aqueous nitric acid solution after the bubbling was collected and diluted to 100 mL (V) with ultrapure water using a female flask. The concentrations of various metal atoms in the diluted aqueous nitric acid solution were measured with an inductively coupled plasma mass spectrometer, and the concentrations (C, unit: g/g) of various metal atoms in the 1-bromo-1,2,2-trifluoroethylene were calculated using the measured values (c1, unit: g/mL) and the following equation.

$$C = \{(c1 \times V) \times (M1/M3)\}/M2$$

[Table 1]

|  | Mn | Co | Ni | Si | Total | Na | K | Mg | Ca | Total | Total of all metal impurities |
|---|---|---|---|---|---|---|---|---|---|---|---|
| Sample 1-1 | 5 | 2 | 1 | 3 | 11 | - | - | - | - | - | 11 |
| Sample 1-2 | 23 | 20 | 16 | 19 | 78 | - | - | - | - | - | 78 |
| Sample 1-3 | 222 | 256 | 165 | 156 | 799 | 214 | 208 | 171 | 178 | 771 | 1570 |
| Sample 1-4 | 442 | 429 | 359 | 389 | 1619 | - | - | - | - | - | 1619 |
| *) Unit of numerical values is ppb by mass. "-" indicates "not measured". | | | | | | | | | | | |

Next, the cylinder A was cooled to about 0°C, forming a liquid phase part and a gas phase part. 100 g of the 1-bromo-1,2,2-trifluoroethylene gas was extracted from an upper outlet, where the gas phase part is present, of the cylinder A, and transferred to the cylinder B in the depressurized state. Further, 10 g of the 1-bromo-1,2,2-trifluoroethylene gas was extracted from the cylinder and transferred to the cylinder B in the depressurized state. Then, the temperature of the cylinder B was raised to room temperature and allowed to stand still for 24 hours. The 1-bromo-1,2,2-trifluoroethylene after still standing is set as Sample 1-2. The 1-bromo-1,2,2-trifluoroethylene gas was extracted from an upper outlet, where the gas phase part is present, of the cylinder B after still standing, and the concentrations of various metal impurities were measured in the same manner as above using an inductively coupled plasma mass spectrometer. The results are shown in Table 1.

Similarly, 100 g of the 1-bromo-1,2,2-trifluoroethylene gas was extracted from the upper outlet, where the gas phase part is present, of the cylinder A, and transferred to the cylinder C in the depressurized state. Further, 100 g of the 1-bromo-1,2,2-trifluoroethylene gas was extracted from the cylinder and transferred to the cylinder C in the depressurized state. Then, the temperature of the cylinder C was raised to room temperature and allowed to stand still for 24 hours. The 1-bromo-1,2,2-trifluoroethylene after still standing is set as Sample 1-3. The 1-bromo-1,2,2-trifluoroethylene gas was extracted from an upper outlet, where the gas phase part is present, of the cylinder C after still standing, and the concentrations of various metal impurities were measured in the same manner as above using an inductively coupled plasma mass spectrometer. The results are shown in Table 1.

Similarly, 100 g of the 1-bromo-1,2,2-trifluoroethylene gas was extracted from the upper outlet, where the gas phase part is present, of the cylinder A, and transferred to the cylinder D in the depressurized state. Further, 200 g of the 1-bromo-1,2,2-trifluoroethylene gas was extracted from the cylinder and transferred to the cylinder D in the depressurized state. Then, the temperature of the cylinder D was raised to room temperature and allowed to stand still for 24 hours. The 1-bromo-1,2,2-trifluoroethylene after still standing is set as Sample 1-4. The 1-bromo-1,2,2-trifluoroethylene gas was extracted from an upper outlet, where the gas phase part is present, of the cylinder D after still standing, and the concentrations of various metal impurities were measured in the same manner as above using an inductively coupled plasma mass spectrometer. The results are shown in Table 1.

(Preparation Example 2)

Samples 2-1 to 2-4 were prepared by performing the same operation as that in Preparation Example 1, except that 1-bromo-1-fluoroethylene was used as fluoroalkene and the temperature of the cylinder A when the gas in the cylinder A was transferred to the cylinder B was set to 10°C. Then, the concentrations of various metal impurities of each sample were measured using an inductively coupled plasma mass spectrometer in the same manner as in Preparation Example 1. The results are shown in Table 2.

[Table 2]

| | Mn | Co | Ni | Si | Total | Na | K | Mg | Ca | Total | Total of all metal impurities |
|---|---|---|---|---|---|---|---|---|---|---|---|
| Sample 2-1 | 6 | 3 | 2 | 3 | 14 | - | - | - | - | - | 14 |
| Sample 2-2 | 25 | 21 | 17 | 17 | 80 | - | - | - | - | - | 80 |
| Sample 2-3 | 219 | 209 | 170 | 172 | 770 | 191 | 167 | 189 | 222 | 769 | 1539 |
| Sample 2-4 | 420 | 417 | 330 | 369 | 1536 | - | - | - | - | - | 1536 |
| *) Unit of numerical values is ppb by mass. "-" indicates "not measured". | | | | | | | | | | | |

(Preparation Example 3)

Samples 3-1 to 3-4 were prepared by performing the same operation as that in Preparation Example 1, except that 2-bromo-3,3,3-trifluoropropene was used as fluoroalkene and the temperature of the cylinder A when the gas in the cylinder A was transferred to the cylinder B was set to 30°C. Then, the concentrations of various metal impurities of each sample were measured using an inductively coupled plasma mass spectrometer in the same manner as in Preparation Example 1. The results are shown in Table 3.

[Table 3]

| | Mn | Co | Ni | Si | Total | Na | K | Mg | Ca | Total | Total of all metal impurities |
|---|---|---|---|---|---|---|---|---|---|---|---|
| Sample 3-1 | 2 | 3 | 3 | 4 | 12 | - | - | - | - | - | 12 |
| Sample 3-2 | 25 | 21 | 18 | 16 | 80 | - | - | - | - | - | 80 |

(continued)

| | Mn | Co | Ni | Si | Total | Na | K | Mg | Ca | Total | Total of all metal impurities |
|---|---|---|---|---|---|---|---|---|---|---|---|
| Sample 3-3 | 217 | 188 | 212 | 251 | 868 | 212 | 210 | 176 | 188 | 786 | 1654 |
| Sample 3-4 | 417 | 428 | 350 | 347 | 1542 | - | - | - | - | - | 1542 |
| *) Unit of numerical values is ppb by mass. "-" indicates "not measured". | | | | | | | | | | | |

(Preparation Example 4)

Samples 4-1 to 4-4 were prepared by performing the same operation as that in Preparation Example 1, except that 1-iodo-1,2,2-trifluoroethylene was used as fluoroalkene and the temperature of the cylinder A when the gas in the cylinder A was transferred to the cylinder B was set to 30°C. Then, the concentrations of various metal impurities of each sample were measured using an inductively coupled plasma mass spectrometer in the same manner as in Preparation Example 1. The results are shown in Table 4.

[Table 4]

| | Mn | Co | Ni | Si | Total | Na | K | Mg | Ca | Total | Total of all metal impurities |
|---|---|---|---|---|---|---|---|---|---|---|---|
| Sample 4-1 | 5 | 3 | 1 | 5 | 14 | - | - | - | - | - | 14 |
| Sample 4-2 | 30 | 22 | 18 | 20 | 90 | - | - | - | - | - | 90 |
| Sample 4-3 | 230 | 218 | 193 | 202 | 843 | 255 | 220 | 193 | 205 | 873 | 1716 |
| Sample 4-4 | 453 | 441 | 354 | 412 | 1660 | - | - | - | - | - | 1660 |
| *) Unit of numerical values is ppb by mass. "-" indicates "not measured". | | | | | | | | | | | |

(Example 1)

The sealed cylinder A was allowed to stand still at 20°C for 30 days, and then the 1-bromo-1,2,2-trifluoroethylene gas was extracted from the gas phase part of the cylinder A and analyzed with gas chromatography and Raman Spectroscopy to quantify the concentrations of decomposition products of the 1-bromo-1,2,2-trifluoroethylene, bromine molecules, and iodine molecules present in Sample 1-1. As a result, no decomposition products, no bromine molecules, and no iodine molecules present were detected.
The measurement conditions of the gas chromatography are as follows.

Gas chromatograph: GC-2014 manufactured by Shimadzu Corporation
Column: carbopackB_1% sp-1000
Injection temperature: 200°C
Column temperature: 100°C
Detector: FID
Detector temperature: 200°C
Carrier gas: helium
Detection limit: 1 ppm by mass

(Examples 2 to 12 and Comparative Examples 1 to 4)

The analysis targets and the analysis results in Examples 2 to 12 and Comparative Examples 1 to 4 are shown in Table 5, in comparison with Example 1. More specifically, the analysis was performed by the same operation as that in Example 1, except for the items shown in Table 5. Table 5 shows ratios (%) of the total area of all the peaks present on the lower molecular weight side relative to the molecular weight of the original fluoroalkene (e.g., 1-bromo-1,2,2-trifluoro-oethylene in Example 1) to the peak area of the original fluoroalkene in the gas chromatography.

[Table 5]

| | Sample/Cylinder | Fluoroalkene | Concentration of decomposition product (Peak area ratio <%>) | Result |
|---|---|---|---|---|
| Ex. 1 | 1-1/A | 1-bromo-1,2,2-trifluoroethylene | Not detected | (-) |
| Ex. 2 | 1-2/B | Same as above | Not detected | (-) |
| Ex. 3 | 1-3/C | Same as above | Not detected | (-) |
| Comp. Ex. 1 | 1-4/D | Same as above | 98 | (+) |
| Ex. 4 | 2-1/A | 1-bromo-1-fluoroethylene | Not detected | (-) |
| Ex. 5 | 2-2/B | Same as above | Not detected | (-) |
| Ex. 6 | 2-3/C | Same as above | Not detected | (-) |
| Comp. Ex. 2 | 2-4/D | Same as above | 26 | (+) |
| Ex. 7 | 3-1/A | 2-bromo-3,3,3-trifluoropropene | Not detected | (-) |
| Ex. 8 | 3-2/B | Same as above | Not detected | (-) |
| Ex. 9 | 3-3/C | Same as above | Not detected | (-) |
| Comp. Ex. 3 | 3-4/D | Same as above | 45 | (+) |
| Ex. 10 | 4-1/A | 1-iodo-1,2,2-trifluoroethylene | Not detected | (-) |
| Ex. 11 | 4-2/B | Same as above | Not detected | (-) |
| Ex. 12 | 4-3/C | Same as above | Not detected | (-) |
| Comp. Ex. 4 | 4-4/D | Same as above | 210 | (+) |
| (-): No decomposition products were detected. (+): Decomposition products were detected. | | | | |

**Claims**

1. A method for storing fluoroalkene,

    the fluoroalkene having a bromine atom or an iodine atom in a molecule,
    the method comprising:

    storing the fluoroalkene in a container, wherein
    the fluoroalkene is at least one type of fluoroethylene represented by a first general formula $C_2H_pF_qX_r$ and, in the first general formula, X represents a bromine atom or an iodine atom, p is an integer of 0 or more and 2 or less, q is an integer of 1 or more and 3 or less, r is an integer of 1 or more and 3 or less, and p + q + r is 4, and fluoropropene represented by a second general formula $C_3H_sF_tX_u$ and, in the second general formula, X represents a bromine atom or an iodine atom, s is an integer of 0 or more and 4 or less, t is an integer of 1 or more and 5 or less, u is an integer of 1 or more and 5 or less, and s + t + u is 6, and
    the fluoroalkene contains or does not contain at least one type among manganese, cobalt, nickel, and silicon as metal impurities, and, when any of the manganese, the cobalt, the nickel, and the silicon is contained, a total concentration of the manganese, the cobalt, the nickel, and the silicon is set to 1000 ppb by mass or less.

2. The method for storing fluoroalkene according to claim 1, comprising:

    storing the fluoroalkene in a container, wherein
    the fluoroalkene further contains or does not contain at least one type among sodium, potassium, magnesium, and calcium as the metal impurities, and, when any of the sodium, the potassium, the magnesium, and the calcium is further contained, a total concentration of the manganese, the cobalt, the nickel, and the silicon and the sodium, the potassium, the magnesium, and the calcium is set to 2000 ppb by mass or less.

3. The method for storing fluoroalkene according to claim 1 or 2, wherein the fluoroalkene is at least one selected from 1-bromo-1,2,2-trifluoroethylene, 1-bromo-1-fluoroethylene, 1-iodo-1,2,2-trifluoroethylene, 1-iodo-1-fluoroethylene, 2-bromo-3,3,3-trifluoropropene, and 2-iodo-3,3,3-trifluoropropene.

4. The method for storing fluoroalkene according to claim 1 or 2, comprising:
   storing the fluoroalkene at a temperature of -20°C or more and 50°C or less.

5. The method for storing fluoroalkene according to claim 1 or 2, wherein a material of the container is manganese steel.

## INTERNATIONAL SEARCH REPORT

| | International application No. |
|---|---|
| | **PCT/JP2023/007363** |

### A. CLASSIFICATION OF SUBJECT MATTER

*C07C 17/38*(2006.01)i; *C07C 21/18*(2006.01)i
FI:   C07C17/38; C07C21/18

According to International Patent Classification (IPC) or to both national classification and IPC

### B. FIELDS SEARCHED

Minimum documentation searched (classification system followed by classification symbols)

C07C17/38; C07C21/18

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| X | JP 2014-534840 A (AMERICAN PACIFIC CORPORATION) 25 December 2014 (2014-12-25) <br> claims, examples | 1-5 |
| X | JP 2017-190311 A (TOSOH F-TECH INC) 19 October 2017 (2017-10-19) <br> claims, examples | 1-5 |
| X | US 2787646 A (ROBERT NEVILLE HASZELDINE) 02 April 1957 (1957-04-02) <br> claims, example 4 | 1-5 |
| X | JP 2001-322955 A (KANTO DENKA KOGYO CO LTD) 20 November 2001 (2001-11-20) <br> claims, examples | 1-5 |
| X | JOURNAL OF THE AMERICAN CHEMICAL SOCIETY. 1956, 78, 59-62 <br> Experimental | 1-5 |
| X | JP 5-331083 A (DAIKIN IND LTD) 14 December 1993 (1993-12-14) <br> claims, examples | 1-5 |

☑ Further documents are listed in the continuation of Box C.       ☑ See patent family annex.

| | |
|---|---|
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **11 April 2023** | **25 April 2023** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

**INTERNATIONAL SEARCH REPORT**

International application No.

**PCT/JP2023/007363**

| C. | DOCUMENTS CONSIDERED TO BE RELEVANT | |
| --- | --- | --- |
| Category* | Citation of document, with indication, where appropriate, of the relevant passages | Relevant to claim No. |
| A | WO 2015/008695 A1 (ASAHI GLASS COMPANY, LIMITED) 22 January 2015 (2015-01-22)<br>paragraph [0019] | 5 |

**INTERNATIONAL SEARCH REPORT**
Information on patent family members

International application No.

**PCT/JP2023/007363**

| Patent document cited in search report | | | Publication date (day/month/year) | Patent family member(s) | | | Publication date (day/month/year) |
|---|---|---|---|---|---|---|---|
| JP | 2014-534840 | A | 25 December 2014 | US | 2013/0146316 | A1 | |
| | | | | claims, examples | | | |
| | | | | WO | 2013/052850 | A1 | |
| | | | | EP | 2763756 | A1 | |
| | | | | CA | 2851255 | A1 | |
| JP | 2017-190311 | A | 19 October 2017 | (Family: none) | | | |
| US | 2787646 | A | 02 April 1957 | (Family: none) | | | |
| JP | 2001-322955 | A | 20 November 2001 | (Family: none) | | | |
| JP | 5-331083 | A | 14 December 1993 | (Family: none) | | | |
| WO | 2015/008695 | A1 | 22 January 2015 | US | 2016/0123534 | A1 | |
| | | | | paragraph [0023] | | | |
| | | | | EP | 3023404 | A1 | |
| | | | | CN | 105358511 | A | |

Form PCT/ISA/210 (patent family annex) (January 2015)

**EP 4 495 095 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014534840 A

- JP 2017190311 A